# EUROPÄISCHE PATENTSCHRIFT

(11) **EP 2 767 286 B1**
(45) Veröffentlichungstag und Bekanntmachung des Hinweises auf die Patenterteilung: **17.01.2018**
(21) Anmeldenummer: 11874043.0
(22) Anmeldetag: 14.10.2011
(51) Int. Cl.: A61K 31/715, A61K 38/05, A61P 1/04

(54) **ORALE PHARMAZEUTISCHE ZUSAMMENSETZUNG ZUR BEHANDLUNG VON ENTZÜNDLICHEN MAGEN-DARM-ERKRANKUNGEN**
ORAL PHARMACEUTICAL COMPOSITION FOR THE TREATMENT OF INFLAMMATORY GASTROINTESTINAL DISEASES
COMPOSITION PHARMACEUTIQUE ORALE POUR TRAITER LES MALADIES INFLAMMATOIRES DE L'APPAREIL DIGESTIF

(43) Veröffentlichungstag der Anmeldung: 20.08.2014
(73) Patentinhaber: Obshestvo S Ogranichennoj Otvetstvennostju "CytoNIR", St. Petersburg 191023 (RU)
(72) Erfinder: MALININ, Vladimir Viktorovich, St.Petersburg 197227 (RU)
(74) Vertreter: Jeck, Anton
(86) Internationale Anmeldenummer: PCT/RU2011/000803
(87) Internationale Veröffentlichungsnummer: WO 2013/055251

(56) Entgegenhaltungen:
- EP-A1- 2 088 154
- WO-A1-96/27368
- WO-A2-2009/064819
- RU-A- 2010 127 706
- RU-C2- 2 165 254
- SU-A1- 1 105 202
- UA-A- 69 730
- T N SAVATEEVA-LYUBIMOVA ET AL: "PHARMACOLOGY OF GASTROINTESTINAL TRACT GASTROPROTECTIVE ACTIVITY OF MODIFIED GLUTAMYL-TRYPTOPHAN DIPEPTIDE ANALOGS AND MELATONIN", EXPERIMENTAL AND CLINICAL PHARMACOLOGY, Bd. 75, Nr. 3, 1. Januar 2012 (2012-01-01) , Seiten 17-21, XP055131149,
- PEREDERII V G ET AL: "EFFICACY OF IMMUNOSTIMULANTS IN NONSPECIFIC ULCEROUS COLITIS", SOVETSKAYA MEDITSINA, Nr. 4, 1990, Seiten 17-19, XP009179317, ISSN: 0038-5077
- TKACH S M ET AL: "Differentiated immunocorrection therapy for unspecific ulcerative colitis", VRACHEBNOE DELO, Bd. 0, Nr. 4, 1992, Seiten 34-36, XP009179316, ISSN: 0049-6804
- USPENSKII V M ET AL: "MORPHOHISTOCHEMICAL ASSESSMENT OF THE EFFICACY OF HYPERBARIC OXYGENATION CYTOCHROME C AND THYMALIN IN THE TREATMENT OF EXPERIMENTAL GASTRIC ULCERS", PATOLOGICHESKAYA FIZIOLOGIYA I EKSPERIMENTAL'NAYA TERAPIYA, Nr. 5, 1990, Seiten 41-42, XP009179319, ISSN: 0031-2991
- 'Klinicheskaya farmakologiya timogena.' POD RED. V. S. SMIRNOVA - SPB Bd. 32, 2004, Seiten 20 - 22, 32, 50-53, XP008170839

## Beschreibung

Die Erfindung betrifft eine pharmazeutische Zubereitung mit Natriumalginat, einem Funktionszusatzstoff und Wasser zur peroralen Behandlung von entzündlichen Magen-Darm-Erkrankungen.

Die Erfindung ist in der Medizin und insbesondere als Peptid-Arzneimittel zur peroralen Verabreichung einsetzbar. Und zwar geht es um pharmazeutische Zubereitungen, die ein Mittel zur peroralen Behandlung von entzündlichen Magen-Darm-Kanal-Erkrankungen enthalten. Es kann eine weite Anwendung in der Gastroenterologie finden.

WO 96/27368 A1 offenbart eine perorale Zusammensetzung zur Behandlung von entzündlichen Magen-Darm-Kanal-Erkrankungen (Gastritis), welche Natriumalginat enthält. Neben Natriumalginat ist auch Kaliumhydrogencarbonat enthalten, um den Säuregehalt des Magensaftes zu regeln. Dem Fachmann ist bekannt, dass anschließend an die pH-Erhöhung als Ergebnis der Wechselwirkung des Magensaftes mit Karbonaten eine sekundäre Säurebildung entsteht, die sogar stärker ist, und zu Sodbrennen und Entzündung der Schleimhaut der Speiseröhre (Ösophagitis) führen kann- was zur wiederholten Einnahme von karbonathaltigen Arzneimitteln zwingt. Dadurch kann die Krankheit statt gelindert sogar verschlimmert werden.

Smirnova V.S. et al. (2004) "Klinicheskaya farmakologiya timogena", POD RED. SPD, Bd 32, Seiten 20-22, 32, 50-53) offenbart eine Zusammensetzung zur Behandlung von entzündlichen Magen-Darm-Kanal-Erkrankungen per Injektion, welche alpha-Glutamyl-Tryptophan (Thymogen) Mononatriumsalz enthält.

Die Wirkungsweise eines Arzneimittels ist von der Darreichungsform abhängig: Der Fachmann unterscheidet zwischen enteralen (z.B. oral, rektal, per Magensonde) und parenteralen Applikationsformen (z. B. durch Injektion).

Es ist bekannt, dass die Beständigkeit von kurzen Peptiden wie beispielsweise des Alpha-Glutamyl-Tryptophan-Dipeptids gegen Magen- und Dünndarm-Enzyme sehr niedrig ist [V.A. Tutelyan und andere. Informationsblatt für experimentelle Biologie und Medizin, 2003, Band 135, Heft 1, S. 4 - 10], wie wir auf Seite 4 Zeilen 1 - 4 der Beschreibung der Anmeldung erwähnt haben. Dass Peptide und kurze Peptide in verschiedenen Abschnitten des Magen-Darm-Kanals unbeständig sind und in einzelne Aminosäuren aufspalten, ist seit langem bekannt, (A. Lehninger. Biochemie, übersetzt aus dem Englischen. "Mir", Moskau, 1974, S. 957; V.K. Antonov. Chemie der Proteolyse. "Nauka", Moskau, 1991, S. 504; G.A. Jarowaja. Bioregelnde Funktion und pathogenetische Rolle der Proteolyse. III. Physiologische Rolle und biochemische Mechanismen des proteolytischen Proteinabbaus; Laboratoriumsmedizin, 2002, Nr. 5, Seiten 39 - 45).

Deshalb wird Thymogen nicht über den Darm (enteral, z.B. peroral) sondern parenteral (in der Regel als Injektion, - siehe bspw. Klinicheskaya et al. (2004) - verabreicht. Durch die Bereitstellung eines enteralen (nämlich peroralen) pharmazeutischen Zusammensetzung von Thymogen (Glutamyl-Tryptophan) zur Behandlung von entzündlichen Magen-Darm-Kanal-Erkrankungen gelang den Erfindern der vorliegenden Anmeldung die Überwindung eines lange bestehenden technischen Vorurteils über das Verhalten kurzer Peptide.

Smirnova et al. (2004) beschreibt den modulierenden Einfluss von synthetischem Glytamyl-Tryptophan-Dipeptid (Thymogen) auf die unspezifische Immunabwehr (Seiten 50-53). Es wird zwar die Injektion von Thymogen zur Behandlung von akuter Pankreatitis beschrieben (Seite 81-87). Die Effizienz des injiziierten Thymogens bestand dabei jedoch "in einer systemischen Wirkung des Präparats auf die Immunität" und **nicht** in einer direkten reparativen Wirkung des Thymogens auf das veränderte Gewebe des Magen-Darm-Kanals.

Die Erkenntnis der vorliegenden Erfindung, dass Thymogen (alpha-Glutamyl-Tryptophan), die Fähigkeit hat, den Säuregehalt des Magensaftes zu regeln ist daher ebenfalls unerwartet.

Ferner ist dem Fachmann bekannt, dass in einer pharmazeutischen Zusammensetzung die Kombination von Wirkstoffen, die woanders getrennt eingesetzt wurden, nicht notwendigerweise zur Addition der Eigenschaften führt; im Gegenteil kann solch eine Kombination dazu führen, dass die Zusammensetzung unwirksam ist oder gar schädliche Nebenwirkungen hat. Nicht umsonst erfordern pharmakologischen Institutionen weltweit die Durchführung von präklinischen und klinischen Prüfungen jeder Neuzusammensetzung, auch wenn sie nur aus seit langem für die medizinische Anwendung zugelassenen Stoffen besteht. Ohne diese Prüfungen kann die Neuzusammensetzung weder in Produktion gehen noch bei Behandlung von Patienten eingesetzt werden. Die Störung der physiologischen Regulationsmechanismen des Verdauungssystems führt in der Regel zur Entwicklung von Polymorbidität, d. h. zur Beteiligung (Einbeziehung) unterschiedlicher Verdauungsorgane am pathologischen Prozess und zur Entwicklung von mehreren Magen-Darm-Kanal-Erkrankungen, die von einem gemeinsamen pathologischen Prozess begleitet sind. Die Pharmakotherapeutik der Erkrankungen von Organen des Verdauungssystems hat ihre Besonderheiten und konkreten Anzeigen je nachdem, welches Organ betroffen ist (Magen, Leber, Pankreas usw.) und welche Krankheitssymptome vorliegen. Die Entwicklung der Polymorbidität ist besonders dem Senioren-Alter eigen. Das setzt einen Sonderansatz an Pharmakotherapeutik voraus. Dieser besteht in der Anwendung von Arzneimitteln, welche den einheitlichen pathogenetischen Mechanismus der gastroenterologischen Krankheiten beeinflussen. Das hängt auch mit der Steigerung der Involutionsprozesse in der Schleimhaut verschiedener Abschnitte des Magen-Darm-Kanals, einer Änderung der Regenerationsintensität und einer Aktivitäts-minderung der Verdauungsfermente bei Alterung des Körpers zusammen [L.N. Valenkevich, A.M. Ugolev. Verdauungssystem // Alterungsbiologie. Handbuch für Physiologie. - L.: Nauka, 1982. - S. 343-369; L.B. Lasebnik, V.N. Drosdov. Krankheiten der Verdauungsorgane bei Senioren. - M.: Anakharsis, 2003. - 208 S.].

Der synthetische Peptid-Immunmodulator Gepon (Thr-Glu-Lys-Arg-Arg-Glu-Thr-Val-Glu-Arg-Glu-Lys-Glu) wird zur peroralen Verabreichung in der Komplextherapie bei älteren Kranken mit nicht durch Heliobacter pylori assoziierte Magengeschwüren benutzt [L.B. Lasebnik und andere. Experimentelle und klinische Gastroenterologie, 2003, Heft 3, S. 17-20].

Die Anwendung von Gepon in der Komplextherapie trug zur Beschleunigung der Regenerationsvorgänge, zur Abkürzung der Vernarbungszeit des ulzerösen Defekts und zur Verbesserung der Kenndaten der zellgebundenen Immunität bei.

Der synthetische Peptid-Immunmodulator Licopid (N-Acetyl- Glukosaminyl- 1 -4-N-Acetylmuramid- L-Alanyl- D-Isoglutamin) wird peroral in der Komplextherapie des chronischen Duodenalulkus durch Heliobacter pylori in Kombination mit chronischer Gastritis angewendet [RU 2252777, 2003; Verfahren zur Behandlung des chronischen Duodenalulkus durch Heliobacter pylori]. Die Komplextherapie unter Einsatz von Licopid zeigt eine hohe Effizienz hinsichtlich der Eradikation von Heliobacter pylori und der Wiederherstellung der struktur- und funktionsbedingten Kenndaten der Magenschleimhaut. Jedoch wurde kein individueller Einfluss von Licopid auf die Pathogenese der Krankheit festgestellt.

Eine bekannte Substanz mit Schutzwirkung bei Magenschäden mit verschiedenen Krankheitsursachen ist Natriumalginat. Natriumalginat wurde in der Dosis von 100 mg/kg den männlichen Wistar- Ratten peroral eine Stunde vor ulzerogener Einwirkung verabreicht. Dabei ließen die Menge und die Größen der destruktiven Bereiche der Magenschleimhaut nach und das Energiegleichgewicht im Magen-gewebe normalisierte sich [E.I. Khasina, A.S. Krivonogova Agrarny Vestnik Urala, 2009, Heft 11 (65), S. 92-94]. Natriumalginat ist Natriumsalz der Algensäure. Dabei handelt es sich um eine Mischung von Polyuronsäuren, die sich aus D-Mannuronsäure- und L-Guluronsäure-Resten zusammensetzen. Das heißt, diese Mischung gehört zu Polysacchariden [A.I. Slivkin VGU Informationsblatt, Serie Chemie, Biologie, 2000, S. 30-46]. Natriumalginat in Form einer Wasserlösung bildet einen Film im Magen, um die Schleimhaut des Magens zu schützen.

Aus dem Stand der Technik ist das Arzneimittel Gaviscon bekannt. Es handelt sich dabei um eine Zubereitung, die eine Wasserlösung von 8-14 Mass./Vol. Natriumalginat und als Funktionszusatzstoff bis zu 5 % Mass./Vol. Kaliumhydrogen-karbonat, bis zu 5% Mass./Vol. Kalziumkarbonat und bis zu 1 % Mass./Vol. Carbomer, inaktiviert durch Natriumhydroxid, darstellt [RU 2173139, I.KI. A61K 9/22, 2001]. Diese Zubereizu 5% Mass./Vol. Kalziumkarbonat und bis zu 1 % Mass./Vol. Carbomer, inaktiviert durch Natriumhydroxid, darstellt [RU 2173139, I.KI. A61K 9/22, 2001]. Diese Zubereitung dient zur Behandlung von Refluxösophagitis, Gastritis, Dyspepsie bzw. Geschwürerkrankung. Die Druckschrift RU 2173139 offenbart, dass infolge der Zusammenwirkung zwischen pharmazeutischer Zubereitung und dem Mageninhalt karbonisierter (mit Kohlensäuregas gesättigter) gelatinöser Schaum oder Filme entstehen, die auf dem Mageninhalt schwimmen. Das ist zweifellos bei Behandlungen von Refluxösophagitis und damit zusammenhängenden unangenehmen Empfindungen nutzvoll. Jedoch, wie es auch bei der Anwendung von anderen den Magensaft-pH-Wert steigernden Präparaten der Fall ist, wird dadurch der Verlauf von entzündlichen und reparativen Prozessen in Organen des Magen-Darm-Kanals wenig beeinflusst.

Es ist Aufgabe der Erfindung, die Effizienz der pharmazeutischen Zubereitungen zur peroralen Behandlung zu steigern.

Die gestellte Aufgabe wird durch die Merkmale des Anspruchs gelöst.

Der genannte Effekt wird wie folgt erreicht.

Die pharmazeutische Zubereitung zur peroralen Behandlung enthält Wasser, Natriumalginat und einen Funktionszusatzstoff. Der erfindungsgemäße Funktionszusatzstoff enthält Dipeptid α-Glutamyl-Tryptophan (Glu-Trp) in Form von Mononatriumsalz mit folgendem Mengenverhältnis in mg, umgerechnet auf eine Dosis von 10 ml:

| | |
|---|---|
| a-Glutamyl-Tryptophan | 0,01 - 0,50 |
| Natriumalginat | 50 - 500 |
| Wasser | Rest |

Die Anwendung von Thymogen-Injektionen ist bei einer Behandlung der akuten Pankreatitis, der postoperativen Peritonitis sowie der Gallensteinkrankheit kompliziert durch akute Cholezystitis bekannt [Herausgegeben von V.S. Smirnov. Klinische Pharmakologie von Thymogen. - S. Petersburg, 2004. - S. 81-87]. Die Effizienz der Anwendung von Thymogen in dieser Arzneiform bestand bei solchen Krankheiten in der Systemeinwirkung des Präparats auf die Immunität. Es wurde kein Einfluss von Thymogen auf den chronischen Verlauf des pathologischen Vorgangs erkannt. Die Thymogen-Effizienz bei Erkrankungen der Magen-Darm-Kanal-Organe bei peroraler Verabreichung wurde nicht erforscht.

Die perorale Anwendung von Thymogen als Bestandteil eines virustötenden Arzneimittels Cytovir erwies sich bei Grippe und akuten viralen Atemwegserkrankungen als effektiv [RU 2165254, 2001 BI Nr. 11]. Es ist nicht möglich, den Einfluss von Thymogen als Bestandteil dieser Zubereitung auf den Zustand der Organe des Magen-Darm-Kanals besonders im Hintergrund einer überschüssigen in Cytovir vorhandenen Askorbinsäure zu erkennen. Darüber hinaus haben die Forschungen der physiologischen Rolle der kurzen Peptide bei der Ernährung gezeigt, dass die Beständigkeit der Peptide gegen Magen- und Dünndarm-Enzyme sehr niedrig ist [V.A. Tutelyan und andere. Informationsblatt für experimentelle Biologie und Medizin, 2003, Band 135, Heft 1, S. 4-10]. Aufgrund des oben Dargelegten scheinen sowohl die Effizienz der peroralen Verabreichung von Thymogen bei Erkrankungen der Magen-Darm-Kanal-Organe als auch die Beständigkeit der angemeldeten Zubereitung gegen Enzyme unerwartet zu sein.

Die erfindungsgemäße Zubereitung wird hergestellt, indem ihre Komponenten im Wasser aufgelöst werden und das gebildete Gel nachher sterilisiert wird.

Die erfindungsgemäße Zubereitung wird hergestellt, indem ihre Komponenten im Wasser aufgelöst werden und das gebildete Gel nachher sterilisiert wird.

Die Zubereitung wurde an Modellen von Geschwürbildung, toxischer Hepatitis und toxischer Pankreatitis sowie im Rahmen der klinischen Praxis bei der Senioren-Behandlung untersucht.

Beispiel 1 Gastroprotektive Aktivität der erfindungsgemäßen pharmazeutischen Zubereitung bei Verabreichung am Modell einer Geschwürbildung.

Die experimentelle Forschung wurde an 50 männlichen Auszucht-Ratten der Linie Sprague-Dawley mit einem Körpergewicht von 180 - 200 g durchgeführt. Vor den Versuchen wurden die Tiere einer 14 Tage langen Quarantäne und Randomisierung unterzogen. Die Tiere wurden unter Vivarium-Bedingungen mit genormter Nahrungsration unterhalten.

Die Tiere wurden in folgende Gruppen aufgeteilt:
- Ratten, die mit einem ulzerogenen Präparat (Indometacin) und Kochsalzlösung (2 ml/kg) behandelt wurden; diese Tiere bilden die Vergleichsgruppe (Referenzgruppe);
- Ratten, die mit einem ulzerogenen Präparat intragastrisch und einem Vergleichspräparat (Sucralfate in der Dosis von 200,0 mg/kg) behandelt wurden;
- Ratten, die mit einem ulzerogenen Präparat intragastrisch und mit der pharmazeutischen Zubereitung (10,0 µg/kg) behandelt wurden;
- Ratten, die mit einem ulzerogenen Präparat intragastrisch und mit der pharmazeutischen Zubereitung (0,1 µg/kg) behandelt wurden.
physiologischen Lösung verabreicht. Die pharmazeutische Zubereitung und das Vergleichspräparat in den oben genannten Mengen wurden im Laufe von 7 Tagen ab dem Zeitpunkt der Simulation des Indometacin-Magengeschwürs (Heilschema) intragastrisch verabreicht. Die Tiere der Referenzgruppe wurden mit Äquivolumen-Mengen des Lösemittels unter entsprechenden Regimes behandelt.

Das Körpergewicht der Tiere wurde im Zeitablauf ermittelt. 24 Stunden nach Abschluss dieser Behandlung wurden die Tiere der Euthanasie mittels prompter Dekapitation unter Blutabnahme unterzogen. Die Mägen wurden entnommen, an der Minorseite aufgeschnitten und mit kalter Kochsalzlösung gewaschen. Die Destruktionszahl wurde makroskopisch mit Hilfe von einem Binokular unter heller Vertikalbeleuchtung ermittelt. Die Destruktionen wurden je nach ihrem Flächeninhalt wie folgt differenziert: Fein (punktförmig, Größe von unter 1 mm), mittelmäßig (rund und streifenförmig, ab 1 mm) und groß (über 1- 2 mm). Das Vorhandensein von Blut an der Destruktionsunterkante wurde anhand der Peroxydase-Reaktion mit Orthodianisidin-Substrat unter Kennfärben der Schleimhaut mit Methylenblau erkannt. Dabei wurde die hämhaltige Oberfläche braun-schwarz im bläulichen Hintergrund der intakten Schleimhaut gefärbt.

Die Verteilung der Tiere in der Gruppe mit Schleimhautfehlern wurde je nach Grad der Ausprägung der makroskopischen Änderungen erfasst. Der Paul's Index (PI) wurde wie folgt berechnet: durchschnittliche Geschwürmenge x % der Tiere mit Geschwüren / 100 %. Die gastroprotektive Aktivität (GA) wurde als PI / PI Verhältnis zwischen der Referenzgruppe und einer Versuchsgruppe berechnet. Das zu untersuchende Präparat wurde als aktiv beurteilt, wenn GA 2 und mehr betrug.

Die statistische Datenverarbeitung wurde anhand der handelsüblichen Software Microsoft Excel durchgeführt. Die Relevanz der Änderungen wurde unter Anwendung des Student t-Tests berechnet.

Es wurde Folgendes festgestellt:
Die Simulation des Magengeschwürs durch Indometacin-Gabe in einer ulzerogenen Menge wird von der Entwicklung von wesentlichen pathologischen Änderungen bei Ratten 3 Tage ab dem Zeitpunkt der Impfung begleitet. Am 7. Tag erreichte die Körpergewichtsabnahme bis zu 30 % gegenüber den Hintergrundwerten. Das hing sowohl mit Blutverlust infolge der Magenblutungen als auch mit der Aktivierung von Katabolismus zusammen. Alle Tiere der Referenzgruppe hatten ausgeprägte Hyperämie und Destruktionserscheinungen in der Magenschleimhaut: Es gab zahlreiche Oberflächen- und Tieferosionen sowie ein Ödem in der Erosions-umgebung. An der Unterkante der meisten Destruktionen wurde häm-positives Material festgestellt (Blut: Oxy- und Methämoglobin infolge des Blutergusses der freigelegten (entblößten) Unterschleimhaut-Kapillare und Hämoglobinoxidation durch Salzsäure des Magensafts). Der Verlauf der Körpergewichtsabnahme ist der Tabelle 1 zu entnehmen.

**Tabelle 1. Einfluss der pharmazeutischen Zubereitung auf den Körpergewichtsverlauf (M±m, g) der Tiere am Modell des Indometacin-Magengeschwürs (m = 10 in jeder Gruppe)**

| Gruppe, Dosis, µg/kg | Beobachtungsdauer | | |
|---|---|---|---|
| | Hintergrund | 3 Tage | 7 Tage |
| Intakt | 196±4 | 195±3 | 200±5 |
| Referenz | 195±2 | 180±3* | 136±5* |
| Sucralfate, 200 mg/kg | 198±3 | 186±4 | 169±3* |
| Pharmazeutische Zubereitung, 10,0 µg/kg | 195±4 | 183±3 | 155±4* |
| Pharmazeutische Zubereitung, 0,1 µg/kg | 200±5 | 191±6 | 177±3** |

| | | | |
|---|---|---|---|
| *- P<0,05 im Vergleich zu Hintergrundwerten; **- P<0,05 im Vergleich zu Referenzgruppe. | | | |

Eine relevante Körpergewichtsabnahme wurde in folgenden Fällen festgestellt:
In der Referenzgruppe und bei Verabreichung der pharmazeutischen Zubereitung in der Dosis von 10,0 µg/kg bei 100 % der Tiere in den jeweiligen Gruppen. Bei der Anwendung der pharmazeutischen Zubereitung in der Dosis von 0,1 µg/kg wurde eine Zunahme des Körpergewichts der Ratten im Vergleich zu den Angaben der Referenzgruppe beobachtet.

Im Hintergrund der Verabreichung des Vergleichspräparats Sucralfate lag eine beachtliche Minderung sowohl der Menge als auch der Fläche von ulzerösen Fehlern vor. Hyperämie der Schleimhaut wurde bei 40 % der Tiere erkannt.

Die Bewertungsergebnisse hinsichtlich des Einflusses der pharmazeutischen Zubereitung auf das Destruktionsausmaß in der Magenschleimhaut der Tiere sind der Tabelle 2 zu entnehmen.

**Tabelle 2. Einfluss der pharmazeutischen Zubereitung auf den Geschwürbildungsprozess bei Tieren am Modell des Indometacin- Magengeschwürs (n = 10 in jeder Gruppe)**

| Gruppe, Dosis, µg/kg | Tiere mit Geschwüren, % | Anzahl der Geschwüre (X ± m) | | | Durchschnitt (X±m) | PI | GA |
|---|---|---|---|---|---|---|---|
| | | Groß | Mittelmäßig | Fein | | | |
| Intakt | 0 | - | - | - | - | - | - |
| Referenz | 100 | 11±2 | 7±1 | 16±3 | 35±3 | 34,7 | - |
| Sucralfate, 200 mg/kg | 100 | 5±2* | 4±1 | 8±2* | 17±4* | 16,9* | 2,05 |
| Pharmazeutische Zubereitung, 10,0 µg/kg | 100 | 4±1* | 7±2 | 11±3 | 21±3* | 21,7* | 1,60 |
| Pharmazeutische Zubereitung, 0,1 µg/kg | 60* | 3±1* | 5±2 | 9±1* | 17±2* | 10,2*^{/}** | 3,40** |
| Sucralfate, 200 mg/kg | 100 | 5±2* | 4±1 | 8±2* | 17±4* | 16,9* | 2,05 |
| Pharmazeutische Zubereitung, 10,0 µg/kg | 100 | 4±1* | 7±2 | 11±3 | 21±3* | 21,7* | 1,60 |
| Pharmazeutische Zubereitung, 0,1 µg/kg | 60* | 3±1* | 5±2 | 9±1* | 17±2* | 10,2*^{/}** | 3,40** |

| | | | | | | | |
|---|---|---|---|---|---|---|---|
| *- P<0,05 im Vergleich zur Referenz; **- P<0,05 im Vergleich zu einem Vergleichspräparat. *- P<0,05 im Vergleich zur Referenz; **- P<0,05 im Vergleich zu einem Vergleichspräparat. | | | | | | | |

Die intragastrische Gabe der pharmazeutischen Zubereitung in verschiedenen Mengen verhinderte die Bildung von Magengeschwüren. Dieser Effekt wurde im größeren Maße im Hintergrund der Verabreichung der Dosis 0,1 µg/kg in Bezug auf die Gesamtanzahl der Magengeschwüre ausgeprägt. In der Dosis von 10,0 µg/kg rief die pharmazeutische Zubereitung eine sichere Minderung der Anzahl von großen Destruktionen hervor und übte keinen Einfluss auf die Menge von mittelmäßigen und kleinen Geschwüren aus.

Beispiel 2 Bewertung der hepatoprotektiven Aktivität der erfindungsgemäßen pharmazeutischen Zubereitung bei Verabreichung am Versuchsmodell der toxischen Hepatitis.

Die Versuche wurden an 40 männlichen Auszucht-Ratten der Linie Sprague-Dawley mit einem Körpergewicht von 200 - 210 g durchgeführt. Jede Versuchsgruppe zählte 10 Tiere. Die zu untersuchenden Präparate wurden den Ratten in Form von Wasserlösung intragastrisch mit einer atraumatischen Metallsonde eingeführt. Die Referenztiere bekamen Reinwasser verabreicht.

Als Vergleichspräparat wurde bei der Untersuchung der hepatoprotektiven Aktivität das Präparat Silibinin in Kapseln, Fabrikat Rosa-Phytopharm (Bulgarien) verwendet. in der gleichen Menge wie das Volumen der hepatoprotektiven Mittel behandelt. Die hepatoprotektive Aktivität wurde 7 und 14 Tage ab Anfang der Behandlung bewertet. Im Blutserum wurden die Aktivität von Aspartat-Amino-transferase (ASAT), Alanin-Aminotransferase (ALT), alkalische Phosphatase sowie der Cholesterinspiegel, Triglyzeridespiegel ermittelt.

Die pathomorphologische Untersuchung umfasste Leichenschau, Makrountersuchung, Verwiegung und histologische Untersuchung der inneren Organe. Die Leichenschau wurde unter direkter Aufsicht eines Pathomorphologen vorgenommen. Nach der Euthanasie wurde die Untersuchung von makro- und mikroskopischen Besonderheiten der Leberstruktur durchgeführt. Zwecks histologischer Untersuchung wurden die Leberstückchen auf dem Mikrotom-Tisch unter Anwendung von flüssigem Kohlendioxid eingefroren und dann im 10%igen Formalinbad fixiert. Die Schnitte wurden mit Sudan IV gefärbt. Die Hepatozytkerne wurden mit Hämatoxylin nachgefärbt.

Die statistische Datenverarbeitung wurde anhand der handelsüblichen Software Microsoft Excel durchgeführt. Die Unterschiede zwischen den Stichproben wurden anhand des Student t-Tests bewertet. Dabei wurde Folgendes festgestellt:
Bei Verabreichung der erfindungsgemäßen pharmazeutischen Zubereitung in den Mengen von 0,1 und 10 µg/kg wurden keine letalen Ausgänge beobachtet. Der Zustand und das Verhalten der Tiere waren normal. In der Gruppe der mit Silibinin behandelten Tiere gab es ebenfalls keine Todesfälle. In der Referenzgruppe waren 3 Ratten von 10 gestorben. Die Angaben sind in der Tabelle 3 aufgeführt.

**Tabelle 3. Einfluss der pharmazeutischen Zubereitung auf die Überlebensfähigkeit der mit toxischer Hepatitis betroffenen Ratten am 14. Tag**

| Versuchsgruppen / Dosis | Todesfälle, abs. (%) |
|---|---|
| TCM (Referenz) | 3/10-(30) |
| TCM+Silibinin (100 mg/kg) | 0/10* |
| TCM+Pharmazeutische Zubereitung (0,1 µg/kg) | 0/10* |
| TCM+Pharmazeutische Zubereitung (10 µg/kg) | 0/10* |

| | |
|---|---|
| * - P<0,05 im Vergleich zur Referenz. | |

Die Aktivität der Leber-Aminotransferasen im Blut gilt als ein Marker von Zytolysesyndrom bei Leberpathologien toxischer Herkunft, und sie änderte sich beachtlich am 7. und 14. Tag der Behandlung bei Ratten der Versuchsgruppen. Ein sicherer Unterschied in der Wirkung der pharmazeutischen Zubereitung wurde bei einer Verabreichung in den Mengen von 0,1 und insbesondere von 10 µg/kg beobachtet. In dieser Hinsicht war die pharmazeutische Zubereitung dem Silibinin in ihrer Wirkung am 14. Behandlungstag überlegen. Die Angaben sind in der Tabelle 4 enthalten.

Die Normalisierung der Aktivität der alkalischen Phosphatase und der Cholesterin- und Triglyzeridespiegel verlief im Hintergrund der Verabreichung der pharmazeutischen Zubereitung in den Mengen von 0,1 und 10 µg/kg. Die 10 µg/kg Dosis der pharmazeutischen Zubereitung hatte einen wesentlichen Einfluss auf die erforschten Kennwerte. Die erfindungsgemäße pharmazeutische Zubereitung stand Silibinin in der Dosis von 100 mg/kg in ihrer Aktivität nicht nach. Die Angaben sind den Tabellen 4 und 5 zu entnehmen.

**Tabelle 4. Einfluss der pharmazeutischen Zubereitung auf die Aktivität der Aminotransferasen und der alkalische Phosphatase im Blut bei toxischer Hepatitis**

| Versuchsgruppen / Dosis, µg/kg | Zu untersuchenden Kenngrößen | | |
|---|---|---|---|
| | ALAT, mMol/Einheiten x L | ASAT, mMol/Einheiten x L | APh, nMol/s x L |
| 7. Behandlungstag | | | |
| Referenz | 2,47±0,14 | 2,10±0,11 | 786±45 |
| Silibinin (100 mg/kg) | 1,56±0,12* | 1,28±0,15* | 469±30* |
| Pharmazeutische Zubereitung (0,1 µg/kg) | 1,73±0,16* | 1,31±0,14* | 455±29* |
| Pharmazeutische Zubereitung (10 µg/kg) | 1,44±0,15* | 1,24±0,17* | 477±23* |

| 14. Behandlungstag | | | |
|---|---|---|---|
| Referenz | 2,20±0,19 | 1,84±0,16 | 604±31 |
| Silibinin (100 mg/kg) | 0,99±0,16* | 0,75±0,11* | 288±17* |
| Pharmazeutische Zubereitung (0,1 µg/kg) | 1,01±0,18* | 0,72±0,12* | 335±22* |
| Pharmazeutische Zubereitung (10 µg/kg) | 0,54±0,10*/** | 0,44±0,17*/** | 221±16*/** |

| | | | |
|---|---|---|---|
| * - P<0,05 im Vergleich zur Referenz; ** - P<0,05 im Vergleich zu dem Vergleichspräparat. | | | |

**Tabelle 5. Einfluss der pharmazeutischen Zubereitung auf Cholesterinspiegel und Triglyzeridespiegel im Blut bei toxischer Hepatitis**

| | | |
|---|---|---|
| Versuchsgruppen | Zu untersuchenden Kenngrößen | |
| | Cholesterin, mMol/ L | Triglyzeride, mMol/ L |

| 7. Behandlungstag | | |
|---|---|---|
| Referenz | 5,06±0,19 | 3,18±0,16 |
| Silibinin (100 mg/kg) | 3,60±0,18* | 2,44±0,12* |
| Pharmazeutische Zubereitung (0,1 µg/kg) | 3,77±0,19* | 2,51±0,10* |
| Pharmazeutische Zubereitung (10 µg/kg) | 3,66±0,16* | 2,38±0,12* |

| 14. Behandlungstag | | |
|---|---|---|
| Referenz | 3,88±0,16 | 2,48±0,19 |
| Silibinin (100 mg/kg) | 1,99±0,12* | 1,48±0,14* |
| Pharmazeutische Zubereitung (0,1 µg/kg) | 2,04±0,16* | 1,33±0,18* |
| Pharmazeutische Zubereitung (10 µg/kg) | 1,46±0,18* | 1,08±0,10*^{/}** |

| | | |
|---|---|---|
| *- P<0,05 im Vergleich zur Referenz; **- P<0,05 im Vergleich zu dem Vergleichspräparat. | | |

Nach Abschluss des Versuchs wurde Euthanasie der Tiere vorgenommen, um den Fettdystrophiegrad der Hepatozyte zu bestimmen. Nach Angaben der Autopsie und der makroskopischen Untersuchung der zu prüfenden Organe wurden keine Unterschiede zwischen den Versuchsgruppen der mit beiden Dosen der pharmazeutischen Zubereitung behandelten Tiere und der mit einem Vergleichs-präparat behandelten Tiere festgestellt.

Die linearen Abmessungen und der Umfang der Leber waren beachtlich vergrößert. Die Leberoberfläche war glatt und unregelmäßig braun bis graubraun gefärbt. An der Schnittstelle war das Lebergewebe dunkelrot mit zahlreichen Blutergüssen. Die Leberkapsel war dünn und durchsichtig. Die Leberkonsistenz (Leberbeschaffenheit) war weich.

Die Milz hatte vergrößerte Abmessungen und Form. Die Milzoberfläche war homogen dunkelkirschbraun und glatt.

Die histologische Untersuchung der Leber (Schnittfärbung - Sudan) ergab Fettdystrophie der zentrolobulären und periportalen Hepatozyte mit unterschiedlichem Ausprägungsgrad. Um den Lipidegehalt halbquantitativ zu bewerten, wird eine Fünfpunkt-Skala benutzt:
1. Mindestgrad der Fettdystrophie: Hepatozyte mit Lipideeinschlüssen nur im periportalen Bereich des Leberläppchens.
2. Schwacher Grad: Hepatozyte mit Lipideeinschlüssen nehmen nur 1/4 - 1/3 der Länge der Lebertrabekel im periportalen Bereich ein.
3. Mäßiger Grad: Ähnliche Hepatozyte nehmen 1/4 - 1/3 der Länge der Lebertrabekel an der Läppchenperipherie ein.
4. Hoher Grad: Hepatozyte mit Fettkügelchen nehmen 1/2 - 2/3 der Länge der Lebertrabekel ein.
5. Höchstgrad: Steatosis des gesamten Leberläppchens, Verschmelzung der Lipozelen.

Die Leber der Ratten der Referenzgruppe enthielt tiefe Leberparenchymschäden in Form von Fett- und Eiweißdystrophie mit Nekrobiose eines Teils der Hepatozyte. Die Fettdystrophie hatte einen diffusen Charakter. Mittelmäßige und große Fettkügelchen füllten das Zytoplasma der meisten Hepatozyte in allen Abschnitten des Leberläppchens aus - dabei handelt es sich um den Höchstgrad der Fettdystrophie.

In der Gruppe der mit Silibinin behandelten Ratten waren fettdystrophiebedingte Änderungen in der Leber der Ratten nur wenig ausgeprägt und hatten einen Herdcharakter. Große Fettkügelchen waren im Zytoplasma von kleinen Gruppen - à 4 bis 5 Hepatozyte im Blickfeld - enthalten. Dabei handelte es sich um den schwachen Grad der Fettdystrophie. Beim Nachfärben durch Hämatoxylin hatten der trabekuläre Aufbau der Leber und die Kerne der Hepatozyte scharfe Konturen.

Die mit pharmazeutischer Zubereitung in der Dosis von 0,1 µg/kg behandelten Ratten hatten weniger ausgebreitete dystrophiebedingte Leberänderungen. Die Fettdystrophie hatte einen Herdcharakter. Ein beachtlicher Teil der Hepatozyte enthielt keine Fetteinschlüsse - hier handelte es sich um den schwachen Grad der Fettdystrophie.

In der Gruppe der mit pharmazeutischer Zubereitung in der Dosis von 10 µg/kg behandelten Ratten wurden dystrophiebedingte Änderungen in einem sehr schwachen Grad erkannt. Es gab praktisch keine dystrophiebedingten Leberänderungen. Feine Fettkügelchen wurden im Zytoplasma der einzelnen Hepatozyte beobachtet - hier lag der Mindestgrad der Fettdystrophie vor. Der trabekuläre Aufbau der Leber und die Kerne der mit Hämatoxylin nachgefärbten Hepatozyte waren scharf zu unterscheiden. Es wurden keine Störungen der tinktoriellen Eigenschaften von Zytoplasma bemerkt. Somit übt die pharmazeutische Zubereitung eine ausgeprägte hepatoprotektive Wirkung am Modell der Hepatopathie toxischer Genese aus. Die pharmazeutische Zubereitung trägt der Wiederherstellung des funktionellen und morphologischen Zustands der Hepatozyte bei.

Beispiel 3 Bewertung der Pankreas-protektiven Aktivität der pharmazeutischen Zubereitung bei therapeutischer Anwendung im Versuchsmodell der toxischen Pankreatitis.

In der Gruppe der mit Silibinin behandelten Ratten waren fettdystrophiebedingte Änderungen in der Leber der Ratten nur wenig ausgeprägt und hatten einen Herdcharakter. Große Fettkügelchen waren im Zytoplasma von kleinen Gruppen - ä 4 bis 5 Hepatozyte im Blickfeld - enthalten. Dabei handelte es sich um den schwachen Grad der Fettdystrophie. Beim Nachfärben durch Hämatoxylin hatten der trabekuläre Aufbau der Leber und die Kerne der Hepatozyte scharfe Konturen.

Die mit pharmazeutischer Zubereitung in der Dosis von 0,1 µg/kg behandelten Ratten hatten weniger ausgebreitete dystrophiebedingte Leberänderungen. Die Fettdystrophie hatte einen Herdcharakter. Ein beachtlicher Teil der Hepatozyte enthielt keine Fetteinschlüsse - hier handelte es sich um den schwachen Grad der Fettdystrophie.

In der Gruppe der mit pharmazeutischer Zubereitung in der Dosis von 10 µg/kg behandelten Ratten wurden dystrophiebedingte Änderungen in einem sehr schwachen Grad erkannt. Es gab praktisch keine dystrophiebedingten Leberänderungen. Feine Fettkügelchen wurden im Zytoplasma der einzelnen Hepatozyte beobachtet - hier lag der Mindestgrad der Fettdystrophie vor. Der trabekuläre Aufbau der Leber und die Kerne der mit Hämatoxylin nachgefärbten Hepatozyte waren scharf zu unterscheiden. Es wurden keine Störungen der tinktoriellen Eigenschaften von Zytoplasma bemerkt. Somit übt die pharmazeutische Zubereitung eine ausgeprägte hepatoprotektive Wirkung am Modell der Hepatopathie toxischer Genese aus. Die pharmazeutische Zubereitung trägt der Wiederherstellung des funktionellen und morphologischen Zustands der Hepatozyte bei.

Beispiel 3 Bewertung der Pankreas-protektiven Aktivität der pharmazeutischen Zubereitung bei im Versuchsmodell der toxischen Pankreatitis.

Die statistische Datenverarbeitung wurde anhand des Softwarepakets Microsoft Excel durchgeführt. Die Unterschiede zwischen den Stichproben wurden anhand des Student t-Tests bewertet.

Die Schutzwirkung der Präparate wurde 72 Stunden und am 5. Tag nach der Simulation der AP anhand der biochemischen Blut- und Pankreaskenndaten im Vergleich mit diesen von den mit nur Sterofundin G-5 behandelten Referenztieren bewertet. (Der Infusionstherapie wurden alle Versuchs- und Referenzratten unterzogen).

In allen Versuchsgruppen der Tiere wurden eine Entwicklung von Bewegungsarmut und ein verstärkter Wasserverbrauch der Ratten 1 Stunde nach der Arginin-Gabe bemerkt. 24 Stunden später wurde bei allen Referenzratten die Aussonderung von schwarzem, flüssigem Darminhalt bemerkt. Die Todesrate der Tiere zu diesem Zeitpunkt betrug 20 %.

48 Stunden später wurde in der Referenzgruppe die verstärkte Ausprägung von AP in Form von Ansammlungen der tastbaren Menge von abdominalem Exsudat und entstandenen Petechien an der Haut unter der Wolldecke beobachtet. Die Todesrate der Tiere nach 48 Stunden betrug 40 %.

72 Stunden später blieben 5 Ratten von 10 in der Referenzgruppe. Das Blutserum dieser Ratten wies folgende Änderungen auf: Drastische stoßartige Steigerung der Aktivität von Amylase (10235,5 U/l), Trypsin (6,65 U/l) und Lipase (19,88 MU/l). Diese Angaben entsprechen einer schweren akuten Pankreasnekrose.

Am 5. Tag verfielen alle Tiere der Referenzgruppe in komatösen Zustand und wurden dem Experiment mittels Euthanasie unter Blutabnahme und Pankreas entnommen.

In den jeweiligen mit pharmazeutischer Zubereitung behandelten Gruppen wurde die Minderung der Letalitätsrate nach 24 Stunden und 72 Stunden und insbesondere bei der Behandlung mit dem Präparat in der Dosis von 0,1 µg/kg beobachtet. Bei den überlebenden Ratten wurden häufige Defäkationen mit schwarzem Darminhalt festgestellt.

Die pharmazeutische Zubereitung trug zu einer Vergrößerung der Menge von überlebenden Tieren mit AP im Vergleich zu der Referenzgruppe bei.

Die experimentelle Therapie von AP mit pharmazeutischer Zubereitung trug zur Minderung der Aktivität der proteolytischen Enzyme im Blutserum bei. Die Angaben sind der Tabelle 6 zu entnehmen.

**Tabelle 6. Einfluss der pharmazeutischen Zubereitung auf die Aktivität der proteolytischen Enzyme im Blut bei akuter Pankreatitis**

| | | | |
|---|---|---|---|
| Versuchsgruppen/ Dosis, µg/kg | Zu untersuchenden Kenngrößen | | |
| | Amylase, MU/l | Trypsin, MU/l | Lipase, MU/l |

| 3. Behandlungstag | | | |
|---|---|---|---|
| Referenz | 10235±356 | 6,65±1,20 | 19,60±1,15 |
| Pharmazeutische Zubereitung (0,1 µg/kg) | 8146±162* | 3,80±0,86*/** | 13,55±1,03*/** |
| Pharmazeutische Zubereitung (10 µg/kg) | 7911±105* | 4,26±0,95* | 17,60±1,12* |

| 5. Behandlungstag | | | |
|---|---|---|---|
| Referenz | 6230±456 | 3,47±0,65 | 6,43±0,52 |
| Pharmazeutische Zubereitung (0,1 µg/kg) | 811±148*/** | 1,79±0,44*/** | 3,35±0,22* |
| Pharmazeutische Zubereitung (10 µg/kg) | 1864±433* | 2,14±0,29* | 2,21±0,16* |

| | | | |
|---|---|---|---|
| * P<0,05 im Vergleich zur Referenz; ** P<0,05 im Vergleich zur Dosis der pharmazeutischen Zubereitung von 10 µg/kg. Anmerkung: † - Tiere wurden aus dem Experiment im komatösen Zustand ausgeschlossen. | | | |

Die Behandlung mit einer pharmazeutischen Zubereitung in den Mengen von 0,1 und 10 µg/kg trug zur Aktivitätssenkung von Amylase, Trypsin und Lipase bei. Dabei konnte ein deutlicher Unterschied in der Wirkung von zwei Dosen ab dem 3. Behandlungstag nachvollzogen werden.

Die antioxidative Wirkung der pharmazeutischen Zubereitung wurde nach dem Einfluss auf die Aktivität der Peroxidase und das Thiol-Disulfidgleichgewicht des Blutserums der Ratten nach 5 Behandlungstagen bewertet.

Bei AP wurde eine ausgeprägte Abnahme des Spiegels der wiederhergestellten sulfhydrylen Blutgruppen und eine Steigerung der Menge von oxidierten Disulfid- Bindungen beobachtet. Dies kennzeichnet einen oxidativen Stress und eine Abschöpfung des Gehalts an Serum-Antioxidans. Die Aktivität der Peroxidase im Blut nahm dabei beachtlich zu.

Die Behandlung mit pharmazeutischer Zubereitung im Hintergrund der Infusionstherapie übte einen positiven Einfluss auf die Kennwerte des Thiol-Disulfid-Gleichgewichts im Blut aus und normalisierte die Aktivität der Peroxidase. Die pharmazeutische Zubereitung in der Dosis von 0,1 µg/kg war der pharmazeutischen Zubereitung in der Dosis von 10 µg/kg und der Referenz an ihrer Wirkung auf die Peroxidase-Aktivität und den Gehalt an Disulfid-Gruppen überlegen. Die entsprechenden Angaben sind der Tabelle 7 zu entnehmen.

**Tabelle 7. Einfluss der pharmazeutischen Zubereitung auf den Zustand des antioxydativen Systems des Bluts bei akuter Pankreatitis**

| Versuchsgruppen/ Dosis, µg/kg | Zu untersuchenden Kenngrößen | | |
|---|---|---|---|
| | Peroxidase, µMol/min. x mg | Sulfhydryle Gruppen, µMol/l | Disulfid-Gruppen, µMol/l |
| 5. Behandlungstag | | | |
| Referenz^{†} | 81,2±7,6 | 258,1±8,0 | 142,5±11,0 |
| Pharmazeutische Zubereitung (0,1 µg/kg) | 23,5±5,4*/** | 341,6±12,4*/** | 62,1±8,5*/** |
| Pharmazeutische Zubereitung (10 µg/kg) | 48,4±6,1* | 306,2±5,9* | 89,0±6,1* |

| | | | |
|---|---|---|---|
| *- P<0,05 im Vergleich zur Referenz; ** - P<0,05 im Vergleich zur Dosis der pharmazeutischen Zubereitung von 10 µg/kg. Anmerkung: † - Tiere wurden aus dem Experiment im komatösen Zustand ausgeschlossen. | | | |

Die Kenndaten des Pankreasgewebes, und zwar der Eiweißgehalt und der Karbonylgruppenspiegel, gelten als Marker und kennzeichnen eine Proteolyse und einen oxidativen Schaden für die Drüse selbst bei Pankreasnekrose. Diese Kennwerte wurden im Hintergrund der Behandlung mit pharmazeutischer Zubereitung (unter Bedingungen der Infusionstherapie mit dem Präparat Sterofundin G-5) normalisiert. Das zeugte von der Schutzwirkung hinsichtlich der Pankreas. Die Angaben sind in der Tabelle 8 enthalten.

Somit stellte die zu untersuchende pharmazeutische Zubereitung die Wiederherstellung der funktionellen Aktivität von Pankreas der an akutem Pankreasnekrose betroffenen Ratten sicher, verhinderte die Proteolyse von Pankreas, verminderte die Fermentemie und übte eine antioxidative und antihypoxische Wirkung im Hintergrund der Infusionstherapie mit Sterofundin G-5 aus.

**Tabelle 8. Einfluss der pharmazeutischen Zubereitung auf den Gesamteiweiß- und Karbonylgruppenspiegel im Pankreas bei akuter Pankreatitis**

| | | |
|---|---|---|
| Versuchsgruppen/ Dosis, µg/kg | Zu untersuchenden Kenngrößen | |
| | Eiweiß, mg/g des Rohgewebes | GKG, nMol/mg von Eiweiß |

| 5. Behandlungstag | | |
|---|---|---|
| Referenz^{†} | 38,0±4,1 | 0,514±0,065 |
| Pharmazeutische Zubereitung (0,1 µg/kg) | 77,6±4,4*/** | 0,276±0,040*/** |
| Pharmazeutische Zubereitung (10 µg/kg) | 54,9±3,6* | 0,359±0,013* |

| | | |
|---|---|---|
| *- P<0,05 im Vergleich zur Referenz; ** - P<0,05 im Vergleich zur Dosis der pharmazeutischen Zubereitung von 10 µg/kg. Anmerkung: † - Tiere wurden aus dem Experiment im komatösen Zustand ausgeschlossen. | | |

Beispiel 4 Effizienz der Anwendung der pharmazeutischen Zubereitung bei Kranken nach Vagotomie mit Pyloroplastik

Die postoperativen Komplikationen sind die Ursache der Bildung der funktionellen Insuffizienz, der Störungen der Ausscheidungsfunktion und motorischen Funktionen verschiedener Abschnitte des Magen-Darm-Kanals.

Die zu untersuchende pharmazeutische Zubereitung wurde zweimal täglich im Laufe von 20 Tagen bei 11 Kranken im postoperativen Zustand nach Duodenalulkus (Vagotomie mit Pyloroplastik) angewendet.

Die Kranken mit verschiedenen Fristen nach der Operation (2 bis 10 Jahre) bemerkten Stuhlstörungen, Bauchauftreibungen, Knurren in der Bauchhöhle, Appetitsabnahme.

Alle Kranken wurden früher symptomatisch und pathogenetisch im Zusammenhang mit dieser Krankheit behandelt.

Die Referenzgruppe zählte 9 Kranke mit gleicher Erkrankung. Diese Patienten wurden konventionell behandelt.

Aufgrund der durchgeführten Untersuchung wurde Folgendes festgestellt: Die Anwendung der pharmazeutischen Zubereitung trug zu einer Appetitsanregung und zur Beseitigung der Dyspepsie-Störungen bei.

Die Gastroduodenoskopie ergab eine Verminderung der entzündlichen Änderungen der Schleimhaut des Pylorusabschnitts des Magens und des Zwölffingerdarms.

Es ist offensichtlich, dass die Normalisierung der Verdauung bei Kranken mit postoperativen Störungen nicht nur mit der regulatorischen Einwirkung der pharmazeutischen Zubereitung auf die Funktion der Zellen der Magen- und Zwölffingerdarmschleimhaut sondern auch mit der Stimulation der Enzym-Aktivität des Duodenalinhalts zusammenhängt.

Die erfindungsgemäße pharmazeutische Zubereitung ruft keine Nebeneffekte, keine Komplikationen und keine Arzneiabhängigkeit hervor.

Beispiel 5 Effizienz der Anwendung der pharmazeutischen Zubereitung bei Kranken, die von chronischer Hepatitis betroffen sind

Die Untersuchung wurde an 15 Kranken im Alter von 27 - 62 Jahren durchgeführt. Die Krankheitsdauer betrug 3 bis 20 Jahre. 87 % der Betroffenen hatten Virushepatitis und 13% toxische Hepatitis in der Anamnese.

Die meisten Patienten hatten Beschwerden über Schmerzen im rechten Hypochondrium sowie allgemeine Schwäche und schnelle Ermüdbarkeit. 65% der Betroffenen bemerkten Dyspepsiestörungen.

Alle Kranken wurden früher symptomatisch und pathogenetisch im Zusammenhang mit der o. g. Erkrankung behandelt.

Die zu untersuchende pharmazeutische Zubereitung wurde zweimal täglich im Laufe von 30 Tagen verabreicht.

Die Referenzgruppe umfasste 12 Kranke mit gleicher Krankheit, die konventionell behandelt wurden.

Im Hintergrund der durchgeführten Behandlung bemerkten 86 % der Kranken ine fehlende Ermüdbarkeit, eine Appetitsverbesserung und eine Steigerung der Arbeitsfähigkeit. Die Intensität des Schmerzsyndroms ließ bei 47 % der Kranken wesentlich nach.

Die größte Aufmerksamkeit wurde während der Untersuchung der Kranken der Bewertung von biochemischen Untersuchungsergebnissen geschenkt. Diese Anga-ben kennzeichnen die Aminotransferase-Aktivität, die Pigment- und die Eiweißbildungsfunktion der Leber.

74% der untersuchten Betroffenen hatten Hyperbilirubinämie, erhöhten Alanin-Aminotransferase-Spielgel (ALT), Aspartat-Aminotransferase-Spiegel (ASAT) und eine geringe Vergrößerung der γ-Globulinfraktion im Bluteiweiß, hauptsächlich durch lgM, was von einer gewissen Aktivität der chronischen Entzündung zeugt.

Die Anwendung der pharmazeutischen Zubereitung trug zur Normalisierung des Bilirubinspiegels sowie zur ALT- und ASAT-Aktivität bei (Tabelle 9).

**Tabelle 9. Einfluss der pharmazeutischen Zubereitung auf biochemische Kenndaten des peripheren Bluts bei Kranken mit chronischer Hepatitis**

| Kenndaten (mMol/l) | Vor Behandlung | Nach Behandlung |
|---|---|---|
| Cholesterin | 5,2±0,5 | 5,1±0,3 |
| Bilirubin | 26,7±1,6 | 21,4±1,2* |
| ASAT | 41,3+2,5 | 31,5±2,4* |
| ALT | 54,2±4,5 | 40,8±2,7* |
| GGT (Gamma-Glutamyltransferase) | 45,4±3,4 | 42,6±4,3 |
| Triglyzeride | 2,5±0,3 | 2,2±0,2 |

| | | |
|---|---|---|
| * - P<0,05 im Vergleich zu den Angaben vor der Behandlung. | | |

Die Untersuchung der Immunglobuline des peripheren Bluts, die ein wesentliches Kriterium für die Aktivität einer Entzündung sind, zeigt einen lgM-Spiegelabbau nach der Behandlung unter Anwendung der erfindungsgemäßen pharmazeutischen Zubereitung (Tabelle 10).

**Tabelle 10. Einfluss der pharmazeutischen Zubereitung auf immunologische Kennwerte bei Kranken mit chronischer Hepatitis**

| Kenngrößen (g/l) | Vor Behandlung | Nach Behandlung |
|---|---|---|
| IgA | 2,15±0,06 | 2,27±0,05 |
| IgM | 3,89±0,06 | 1,40±0,01* |
| IgC | 14,6±1,3 | 13,2±0,4 |

| | | |
|---|---|---|
| * - P<0,05 im Vergleich zum Kennwert vor der Behandlung. | | |

Somit zeugen die ermittelten Ergebnisse davon, dass die Anwendung der pharmazeutischen Zubereitung bei von chronischer Hepatitis betroffenen Patienten eine Normalisierung von metabolischen Prozessen in der Leber sowie eine Aktivitätsabnahme der Entzündung fördert und den Hepatozytentod verhindert.

Die erfindungsgemäße pharmazeutische Zubereitung ruft keine Nebeneffekte, keine Komplikationen und keine Arzneiabhängigkeit hervor.

Beispiel 6 Effizienz der Anwendung der pharmazeutischen Zubereitung bei Kranken, die von chronischer Pankreatitis betroffen sind

Die pharmazeutische Zubereitung wurde zweimal täglich im Laufe von 30 Tagen 10 Patienten im Alter von 56 - 75 Jahren mit einer Diagnose chronischer latenter Pankreatitis verabreicht.

Die Referenzgruppe umfasste 9 Kranke mit gleicher Krankheit, denen eine konventionelle Behandlung verordnet wurde.

Die Beschwerden der von chronischer Pankreatitis betroffenen Patienten schlossen eine Appetitlosigkeit, ein Aufstoßen, einen Meteorismus, ein Bauchknurren und Stuhlstörungen ein.

Es wurde festgestellt, dass die Anwendung der pharmazeutischen Zubereitung bei Kranken mit chronischer Pankreatitis zur Appetitsverbesserung sowie zur Minderung der Häufigkeit von Dyspepsiestörungen beitrug.

Im Rahmen der Laboruntersuchung des Duodenalinhalts wurde eine initiale Aktivitätsabnahme bei Pankreasfermenten festgestellt (Tabelle 11).

**Tabelle 11. Einfluss der pharmazeutischen Zubereitung auf die Aktivität der Verdauungsenzyme bei Kranken mit chronischer Pankreatitis**

| Kenngrößen | Vor Behandlung | Nach Behandlung |
|---|---|---|
| Trypsin, mMol/l | 2,14±0,09 | 3,2+0,1* |
| α-Amylase, kg/(h • l) | 5,2±0,4 | 7,1±0,2* |

| | | |
|---|---|---|
| * - P<0,05 im Vergleich zum Kennwert vor der Behandlung. | | |

Als Ergebnis der Anwendung der erfindungsgemäßen pharmazeutischen Zubereitung wurde eine sichere Steigerung der Aktivität von Pankreas-Enzymen festgestellt. Das korreliert mit einer Verbesserung der klinischen Symptomatik.

Die erfindungsgemäße pharmazeutische Zubereitung ruft keine Nebeneffekte, keine Komplikationen und keine Arzneiabhängigkeit hervor.

Somit zeugen die bei der experimentellen und klinischen Forschung erkannten pharmakologischen Eigenschaften der pharmazeutischen Zubereitung von ihrer positiven Einwirkung auf die einheitlichen pathogenetischen Mechanismen der gastroenterologischen Krankheiten und die Zukunftsfähigkeit der Anwendung zur Behandlung von entzündlichen Magen-Darm-Kanal-Erkrankungen.

### Gewerbliche Anwendbarkeit

Die perorale pharmazeutische Zubereitung kann in Arzneimittelbetrieben hergestellt werden, welche sich auf die Herstellung von peroral anwendbaren Medikamenten spezialisiert haben.

**Tabelle 11. Einfluss der pharmazeutischen Zubereitung auf die Aktivität der Verdauungsenzyme bei Kranken mit chronischer Pankreatitis**

| Kenngrößen | Vor Behandlung | Nach Behandlung |
|---|---|---|
| Trypsin, mMol/l | 2,14±0,09 | 3,2+0,1* |
| α-Amylase, kg/(h • l) | 5,2±0,4 | 7,1±0,2* |

| | | |
|---|---|---|
| * - P<0,05 im Vergleich zum Kennwert vor der Behandlung. | | |

Als Ergebnis der Anwendung der erfindungsgemäßen pharmazeutischen Zubereitung wurde eine sichere Steigerung der Aktivität von Pankreas-Enzymen festgestellt. Das korreliert mit einer Verbesserung der klinischen Symptomatik.

Die erfindungsgemäße pharmazeutische Zubereitung ruft keine Nebeneffekte, keine Komplikationen und keine Arzneiabhängigkeit hervor.

Somit zeugen die bei der experimentellen und klinischen Forschung erkannten pharmakologischen Eigenschaften der pharmazeutischen Zubereitung von ihrer positiven Einwirkung auf die einheitlichen pathogenetischen Mechanismen der gastroenterologischen Krankheiten und die Zukunftsfähigkeit der Anwendung zur peroralen Behandlung von entzündlichen Magen-Darm-Kanal-Erkrankungen.

### Gewerbliche Anwendbarkeit

Die pharmazeutische Zubereitung zur peroralen Behandlung kann in Arzneimittelbetrieben hergestellt werden, welche sich auf die Herstellung von peroral anwendbaren Medikamenten spezialisiert haben.

## Patentansprüche

1. Pharmazeutische Zubereitung mit Natriumalginat, einem Funktionszusatzstoff und Wasser zur Anwendung in der peroralen Behandlung von entzündlichen Magen-Darm-Kanal-Erkrankungen,
**dadurch gekennzeichnet,**
**dass** sie α-Glutamyl-Tryptophan Dipeptid in Form von Mononatriumsalz als Funktionszusatzstoff mit folgendem Mengenverhältnis in mg umgerechnet auf eine Dosis von 10 ml enthält:
| | |
|---|---|
| α-Glutamyl- Tryptophan | 0,01 - 0,50 |
| Natriumalginat | 50 - 500 |
| Wasser | Rest |

## Claims

1. A pharmaceutical preparation comprising sodium alginate, a functional additive and water for use in the oral treatment of inflammatory gastrointestinal diseases,
**characterised in that**
it contains α-glutamyl-tryptophan dipeptide in the form of monosodium salt as functional additive with the following quantity ratio in mg converted to a dose of 10 ml:
| | |
|---|---|
| α-glutamyl-tryptophan | 0.01-0.50 |
| sodium alginate | 50-500 |
| water | remainder |

## Revendications

1. Préparation pharmaceutique contenant de l'alginate de sodium, un adjuvant fonctionnel et de l'eau, destinée à être utilisée dans le traitement peroral des maladies inflammatoires de l'appareil digestif,
**caractérisée en ce qu'**elle
contient un dipeptide α-glutamyl-tryptophane sous forme de sel monosodique en tant qu'adjuvant fonctionnel, ayant les proportions suivantes en mg rapportées à une dose de 10 ml :
| | |
|---|---|
| α-glutamyl-tryptophane | 0,01 à 0,50 |
| Alginate de sodium | 50 à 500 |
| Eau | le reste |
